Europäisches Patentamt

European Patent Office

Office européen des brevets

(19)

(11) Numéro de publication : **0 087 192**
**B1**

(12) **FASCICULE DE BREVET EUROPÉEN**

(45) Date de publication du fascicule du brevet :
**30.12.86**

(21) Numéro de dépôt : **83200228.1**

(22) Date de dépôt : **15.02.83**

(51) Int. Cl.⁴ : **C 07 C 59/01**, A 61 K 31/19,
A 61 K 31/205

(54) Compositions pharmaceutiques contenant de l'acide 3-hydroxybutanoïque ou un sel dérivé de cet acide et composés dérivés de l'acide 3-hydroxybutanoïque utilisables comme médicament.

(30) Priorité : **23.02.82 FR 8203100**

(43) Date de publication de la demande :
**31.08.83 Bulletin 83/35**

(45) Mention de la délivrance du brevet :
**30.12.86 Bulletin 86/52**

(84) Etats contractants désignés :
**AT BE CH DE FR GB IT LI LU NL SE**

(56) Documents cités :
**FR-A- 2 369 343**
**US-A- 2 101 097**
**US-A- 3 830 931**
**US-A- 3 879 537**
**US-A- 3 920 835**
**CHEMICAL ABSTRACTS, vol. 72, no. 5, 2 février 1970,
page 45, no. 18825u, Columbus Ohio (US); D.E.HELL-
MANN et al.: "Antilipolytic effects of beta-hydroxybutyrate"**

(73) Titulaire : **SOLVAY & Cie (Société Anonyme)**
**Rue du Prince Albert, 33**
**B-1050 Bruxelles (BE)**

(72) Inventeur : **Lammerant, Jacques**
**Avenue de la Vecquée, 36**
**B-5000 Namur (BE)**
Inventeur : **Kolanowski, Jaroslaw**
**Rue du Long Chêne, 87**
**B-1970 Wezembeek-Oppem (BE)**

## Description

La présente invention concerne des compositions pharmaceutiques pour la protection métabolique du myocarde en cas d'affections telles que les cardiomyopathies d'origine ischémique ou non-ischémique.

Il est connu par le document US-A-2 101 097 que l'acide 3-hydroxybutanoïque peut être administré oralement et que l'isomère lévogyre engendre une activité bactériostatique sur les urines excrétées.

Il est également connu par le document US-A-3 920 835 que l'acide 3-hydroxybutanoïque peut être utilisé dans des usages externes pour des traitements de la peau tels que l'acné.

De son côté le document FR-A-2 369 343 divulgue que l'acide 3-hydroxybutanoïque et le sel de sodium de cet acide ont une fonction physiologique de nutrition par voie parentérale et peuvent être utilisés pour remplacer partiellement ou entièrement les composés gras pour ce qui concerne l'apport en calories pour le métabolisme intermédiaire.

Enfin, il est encore connu par le document US-A-3 830 931 que la carnitine ou acide β-hydroxy-γ-triméthylaminobutanoïque ou son sel avec le chlorure d'hydrogène correspondant permettent de traiter certaines affections du muscle cardiaque.

On a maintenant trouvé de nouvelles compositions et de nouveaux composés permettant la protection métabolique de myocarde.

Les compositions selon l'invention contiennent un sel dérivé de l'acide 3-hydroxybutanoïque.

Les composés selon l'invention sont des sels dérivés de l'acide 3-hydroxybutanoïque et d'une base organique azotée telle qu'un acide aminé.

Les sels présents dans les compositions selon l'invention peuvent être dérivés de n'importe quelle base inorganique ou organique.

Ce peuvent être notamment des sels d'ammonium ou des sels métalliques ; dans ce cas, ils peuvent être dérivés de métaux des groupes Ia, IIa et IIb du tableau périodique des éléments tels que le zinc, le calcium et, plus particulièrement, le sodium. Ce peuvent être des sels dérivés de l'acide 3-hydroxybutanoïque et de n'importe quelle base organique azotée telle que les sels dérivés d'acides aminés.

Les composés selon l'invention peuvent être des sels dérivés de l'acide 3-hydroxybutanoïque et d'un acide aminé. Parmi ceux-ci, les sels dérivés d'acides aminés d'origine naturelle et plus encore les sels dérivés d'acides aminés d'origine naturelle de forme isomérique L. De bons résultats peuvent être obtenus avec les sels dérivés d'acides aminés d'origine naturelle de forme isomérique L comprenant au moins deux fonctions azotées par fonction carboxylique et, plus particulièrement, avec les sels dérivés de la L-lysine, de L-histidine et de L-arginine. Les meilleurs résultats ont été obtenus avec les sels dérivés de L-arginine. Les composés décrits ci-avant sont préférés pour les compositions pharmaceutiques selon l'invention.

L'acide 3-hydroxybutanoïque présent dans les compositions selon l'invention et dont sont dérivés les sels présents dans les compositions selon l'invention ainsi que les composés selon l'invention peuvent être de diverses formes isomériques telles que les formes L(+) et racémiques. De préférence, cependant, cet acide présente la forme isomérique D(—). L'acide 3-hydroxybutanoïque peut êre obtenu par n'importe quelle méthode connue de synthèse telle que les synthèses chimiques ou biochimiques directes. L'acide D(—) 3-hydroxybutanoïque peut être obtenu avantageusement par dépolymérisation de polymères naturels extraits de biomasses selon le procédé décrit dans la demande de brevet européen EP-A-0 043 620 (SOLVAY & Cie).

Les sels de l'acide 3-hydroxybutanoïque peuvent être préparés par toute synthèse organique appropriée. En ce qui concerne les sels dérivés de l'acide 3-hydroxybutanoïque de forme isomérique D(—) ou L(+) et d'une amine et plus particulièrement d'acides aminés d'origine naturelle, le procédé préféré consiste à faire réagir l'acide 3-hydroxybutanoïque avec l'acide aminé d'origine naturelle dans un solvant approprié tel que l'eau et à une température comprise entre 20 °C et 80 °C et de préférence comprise entre 30 et 50 °C ; pour ce faire on met généralement en œuvre des milieux contenant l'acide 3-hydroxybutanoïque et l'acide aminé d'origine naturelle en quantités équimolaires ou légèrement excédentaires à la stœchiométrie en acide 3-hydroxybutanoïque sous forte agitation et à une pression voisine de la pression atmosphérique. Les concentrations des milieux mis en œuvre sont comprises entre 0,5 et 8 molaires en acide 3-hydroxybutanoïque et en acide aminé ; de préférence cette concentration est située entre 1 et 6 molaires. La fin de la réaction est caractérisée par un épaississement ou une prise en masse du mélange réactionnel. La récupération des sels peut ensuite être réalisée par les méthodes habituelles telles que l'évaporation du solvant avec obtention des sels sous forme solide ; ceux-ci peuvent éventuellement subir ensuite une ou plusieurs recristallisations.

Les composés et compositions selon l'invention peuvent être utilisés comme médicaments et notamment pour le traitement et la prévention en médecine humaine et en médecine vétérinaire d'affections métaboliques du myocarde. Dans ce cas, ils sont de préférence formulés pour servir comme moyen pharmaceutique destiné à un traitement interne. Par ailleurs ces composés et compositions peuvent également être utilisés pour traiter ou prévenir d'autres affections telles que les maladies résultant de déficiences dans le métabolisme des corps cétoniques ou l'épilepsie ; dans ce cas on préfère travailler avec des composés ou compositions à base de sels dérivés d'acide 3-hydroxybutanoïque et de zinc et tout particulièrement avec les sels dérivés des acides aminés précités.

Les compositions selon l'invention peuvent être constituées uniquement de sels dérivés d'acides 3-hydroxybutanoïque mais contiennent habituellement des additifs de formulation permettant de les administrer commodément, par exemple sous forme de poudres, de tablettes, capsules, dragées, pilules, granulés, suppositoires, gélules, ampoules, sirops, émulsions, solutions ou suspensions. Ces additifs peuvent être des supports ou des solvants non toxiques habituellement utilisés en pharmacie tels que l'eau, les solvants organiques de type paraffinique, et les huiles végétales et alcooliques aliphatiques, des sels minéraux et des charges habituels, des agents émulsionnants du type des polyoxyalkylènes ou des sulfonates aromatiques ou aliphatiques, des dérivés de la cellulose, de l'amidon, des agents dispersants, des agents lubrifiants comme les sels d'acide stéarique ou le talc, des liants et des agents édulcorants ou aromatisants habituels.

Les compositions pharmaceutiques selon l'invention contiennent en général entre 1 % et 99 % en poids de un ou plusieurs acides 3-hydroxybutanoïques ou sels dérivés de ces acides. Elles peuvent être administrées pour traiter ou prévenir les troubles métaboliques du myocarde de tous les mammifères mais en particulier des patients humains sous forme d'une ou de plusieurs « unités de dosage » ou « doses d'administration » en quantité pharmaceutiquement efficace.

Par « unité de dosage » ou « dose d'administration » on entend une dose unitaire qui peut être administrée à un patient et peut être facilement manipulée et conditionnée, en restant sous forme d'une dose unitaire physiquement stable, contenant l'ingrédient actif soit seul, soit en mélange avec des diluants ou supports pharmaceutiques solides ou liquides.

Les compositions selon l'invention peuvent être formulées notamment pour être administrées par voie orale, par voie rectale, par injection intramusculaire ou en perfusion intraveineuse.

Lorsqu'elles sont destinées à être administrées par voie orale sous forme solide, les compositions peuvent contenir jusqu'à 5 g d'acide 3-hydroxybutanoïque par dose d'administration. Habituellement, les doses contiennent entre 0,1 g et 3 g, et de préférence entre 0,5 g et 2 g, d'acide 3-hydroxybutanoïque ou d'une quantité équivalente de sel.

Les compositions selon l'invention se présentant sous forme liquide et destinées à être administrées par voie orale peuvent être des solutions, des émulsions ou des suspensions, sirupeuses ou non. Elles contiennent habituellement une quantité d'acide 3-hydroxybutanoïque ou de sel dérivé de celui-ci qui est généralement comprise entre 1 g et 750 g, et de préférence entre 10 et 500 g, par litre de liquide.

Lorsqu'elles sont destinées à être administrées par injection intramusculaire, les compositions selon l'invention peuvent être formulées, de façon stérile, sous forme concentrée ou prête à l'emploi, de manière à contenir habituellement au moins 0,05 % en poids, et de préférence entre 0,1 % et 30 % en poids, d'acide 3-hydroxybutanoïque, la concentration maximale étant fixée par l'isotonicité avec le sérum sanguin. Dans le cas du sel de sodium de l'acide D(—)-3-hydroxybutanoïque, les concentrations préférées des liquides prêts pour l'injection se situent entre 1 g et 20 g par litre de liquide. Lorsqu'il s'agit du sel d'arginine de l'acide D(—)-3-hydroxybutanoïque, les concentrations préférées des liquides prêts pour l'injection sont situées entre 1 et 45 g par litre de liquide. En cas d'utilisation de formulations hypotoniques, l'isotonicité peut être réalisée par incorporation d'agents connus à cet effet tels que le chlorure de sodium ou le dextrose. Elles peuvent également contenir des diluants stériles tels que l'eau, les huiles non volatiles, les polyéthylèneglycols, la glycérine et les polypropylèneglycols, des agents antibactériens tels que l'alcool benzylique, des antioxydants tels que l'acide ascorbique et le bisulfite de sodium, des agents chélatants et des agents tampons tels que des acétates, des citrates ou des phosphates, physiologiquement compatibles.

Les formulations destinées à être administrées par injection intramusculaire peuvent être conditionnées de toutes les façons habituelles et se présenter notamment en ampoules ou seringues à jeter après usage, en verre ou en plastique.

Les compositions selon l'invention destinées à être administrées par perfusion intraveineuse peuvent également être formulées selon les techniques et avec les additifs habituellement utilisés dans ce domaine. Les concentrations de ce type de formulations en acide 3-hydroxybutanoïque ou en sel dérivé de celui-ci sont en général supérieures à 0,001 % en poids. Dans le cas du sel de sodium de l'acide D(—)-3-hydroxybutanoïque, la concentration utilisée habituellement varie entre 0,05 g et 20 g, et de préférence entre 0,1 et 15 g, par litre de liquide. Dans le cas du sel dérivé d'arginine de l'acide D(—)-3-hydroxybutanoïque, ces concentrations varient habituellement entre 0,05 et 45 g, et de préférence entre 0,1 et 35 g, par litre de liquide.

Les compositions pharmaceutiques selon l'invention peuvent contenir d'autres principes actifs vis-à-vis d'affections du système cardiaque ou de la circulation sanguine. Parmi ceux-ci, on peut citer à titre d'exemples, les hétérosides cardiotoniques, les glucosocorticoïdes, les bêta-bloquants, les antagonistes calciques, les agents permettant d'abaisser la tension artérielle, les anti-arythmiques et les anticoagulants pour autant qu'il y ait compatibilité.

Les compositions pharmaceutiques selon l'invention sont utilisables pour la protection métabolique du myocarde, dans des traitements prophylactiques ou curatifs notamment à l'occasion d'activités physiques intenses, tels que des activités sportives ou des actes professionnels, imposant un surcroît de consommation d'oxygène au cœur réputé normal, dans les cardiomyopathies d'origine non ischémique, dans les cardiomyopathies d'origine ischémique telles que l'angine de poitrine ou l'infarctus du

3

myocarde, dans l'ischémie du myocarde résultant d'un effondrement de la pression de perfusion coronaire tel qu'observé dans les états de choc circulatoire par hémorragie et d'une façon générale, dans toute situation de décharge adrénergique telle qu'observée dans les états de stress ou de traumatismes sévères.

Le mode d'action des compositions selon l'invention se révèle particulièrement original. En effet, jusqu'à présent, il était habituel de pallier les insuffisances d'apport d'oxygène au myocarde dues à son ischémie en réduisant l'effort à produire. Or, les essais sur chiens effectués avec les compositions selon l'invention ont montré que les indices de rendement mécanique du cœur, tels le rapport entre la pression aortique moyenne et la consommation en oxygène du myocarde, sont améliorés. Les doses à administrer journalièrement peuvent varier dans de larges limites mais sont en général calculées de façon à représenter entre 0,001 et 1 g d'acide 3-hydroxybutanoïque ou de sel dérivé de celui-ci par kg de poids ; la dose journalière préférée se situe entre 0,005 et 0,5 g/kg.

L'invention est illustrée par les exemples suivants.

Exemple 1

Préparation du sel de l'acide D(—)-3-hydroxybutanoïque et de L(+)-arginine

Dans un réacteur cylindrique, muni d'une double enveloppe permettant de refroidir ou chauffer le réacteur, de 30 cm de haut et 11 cm de diamètre et muni d'un agitateur à 4 pales recouvertes de fluorure de polyvinylidène, on introduit 1 kg d'une solution aqueuse contenant 593 g (5,7 moles) d'acide D(—)-3-hydroxybutanoïque. Le tout est agité violemment et on introduit 960 g de L(+)-arginine en 5 minutes. Lorsque la température du mélange réactionnel atteint 45° on refroidit le mélange par circulation d'eau à 20 °C dans la double enveloppe tout en maintenant le mélange sous agitation. On observe un épaississement progressif de la suspension avec finalement prise en masse du mélange, 15 minutes après la fin de l'introduction de la L(+)-arginine. Le solide est alors récupéré et séché en étuve à vide à 50 °C jusqu'à poids constant. On récupère finalement un sel de L(+)-arginine de l'acide D(—)-3-hydroxybutanoïque dont le point de fusion est compris entre 100 et 105 °C et le pouvoir rotatoire, mesuré sur une solution aqueuse d'une concentration de 4,60 g/100 ml, à 27 °C : $[\alpha]_D^{27\,°C} = 2,32°$.

Exemple 2

Essai 1

Cet essai est effectué à titre comparatif sur six chiens, repertoriés A, B, C, D, E et F, dont le sexe et le poids sont mentionnés au Tableau I ci-après, anesthésiés par injection sous-cutanée de

Tableau I

| Chiens | Sexe | poids en kg |
|--------|------|-------------|
| A | M | 24,5 |
| B | M | 29 |
| C | M | 38 |
| D | F | 25 |
| E | F | 24 |
| F | M | 26,5 |

2 mg/kg de morphine et administration de 30 mg/kg de nembutal, et maintenus sous respiration artificielle au moyen d'une pompe de Starling. On mesure la pression aortique, la consommation en oxygène du myocarde, la pression partielle en oxygène du sang veineux coronaire, le débit coronaire, le pH aortique ainsi que la consommation par le myocarde de lactase, de glucose, des acides gras libres et du 3-hydroxybutanoate. Les valeurs moyennes observées sont mentionnées au Tableau III ci-après.

Essai 2

Cet essai est également effectué à titre comparatif.

Les six chiens de l'essai 1 maintenus dans les conditions d'anesthésie précitées sont soumis à une stimulation adrénergique par infusion intraveineuse de noradrénaline aux doses et pendant les durées mentionnées au Tableau II ci-après.

# 0 087 192

Tableau II

| Chien | Quantité de nor-adrénaline en ng/kg.min | Durée en min |
|---|---|---|
| A | 400 | 15 |
| B | 1 000 | 15 |
| C | 400 | 20 |
| D | 400 | 10 |
|  | 800 | 10 |
| E | 400 | 10 |
|  | 800 | 10 |
|  | 1 600 | 10 |
| F | 1 000 | 15 |

Les mêmes paramètres qu'à l'essai 1 sont mesurés et les valeurs moyennes de ces mesures sont mentionnées au Tableau III ci-après.

Essai 3

Cet essai est effectué en mettant en œuvre une composition selon l'invention.

On répète l'essai 2 sur les mêmes chiens sauf que les animaux reçoivent une infusion intraveineuse du sel de sodium d'acide D(—)-3-hydroxybutanoïque à raison de 80 micromoles par kg de poids corporel et par minute.

Les valeurs moyennes des paramètres mesurés sont mentionnées au Tableau III ci-après.

Tableau III

| Paramètre | Essai | | |
|---|---|---|---|
| | 1 | 2 | 3 |
| Pression aortique en mm Hg | 106 | 122 | 136 |
| Consommation en oxygène du myocarde en ml/100 g.min | 8,1 | 13,8 | 12,5 |
| Pression partielle du sang veineux coronaire en oxygène en mm Hg | 22,9 | 24,4 | 24,4 |
| Débit coronaire en ml/100 g.min | 82 | 120 | 131 |
| pH aortique | 7,401 | 7,338 | 7,392 |
| Concentration plasmatique artérielle en 3-hydroxy-butanoate en m. °/$_{oo}$ | 0,02 | 0,13 | 5,97 |

5

(Suite)

| Paramètre | Essai | | |
|---|---|---|---|
| | ·1 | 2 | 3 |
| Consommation par le myocarde | | | |
| - lactate en ʮ mol/100 g.min | 18 | 0 | 0 |
| - glucose en ʮ mol/100 g.min | 8 | 0 | 0 |
| - acides gras libres en ʮ mol/100 g.min | 6 | 16 | 10 |
| - 3-hydroxybutanoate en ʮ mol/100 g.min | 0,2 | 2,3 | 73 |

La comparaison des résultats du Tableau III montre que l'administration du sel de sodium de l'acide D(—)-3-hydroxybutanoïque entraîne la réduction de la consommation propre en oxygène du myocarde soumis à une décharge adrénergique (comparaison des essais 2 et 3) et l'augmentation du rapport de la pression aortique à la consommation en oxygène qui devient plus élevé (comparaison des essais 2 et 3) et se rapproche des valeurs mesurées au repos (comparaison des essais 1 et 3).

Les résultats montrent également que l'assimilation des acides gras libres baisse au profit du 3-hydroxybutanoate (comparaison des essais 2 et 3) et se rapproche des valeurs observées avec le myocarde non stimulé (comparaison des essais 1 et 3).

Enfin, l'acidose métabolique déclenchée par la décharge adrénergique est corrigée (comparaison des essais 1, 2 et 3).

## Exemple 3

Essais 4 à 6

Sur un chien mâle de 28 kg, on répète les essais décrits ci-avant mais avec une stimulation effectuée à l'aide d'une infusion intraveineuse de noradrénaline de 200 ng/kg·min pendant 25 min et une administration intraveineuse de 40 micromoles/kg·min de sel de L-arginine de l'acide D(—)-3-hydroxybutanoïque de l'exemple 1. Les paramètres mesurés au cours de l'essai (essai 6) et au cours d'essais de comparaison réalisés en l'absence de stimulation et de traitement (essai 4) et en effectuant la stimulation mais pas le traitement (essai 5) sont mentionnés au Tableau IV ci-après.

Tableau IV

| Paramètre | Essai | | |
|---|---|---|---|
| | 4 | 5 | 6 |
| Pression aortique en mm Hg | 94 | 132 | 94 |
| Consommation en oxygène du myocarde en ml/100 g.min | 8,9 | 13,4 | 6,9 |
| Pression partielle du sang veineux coronaire en oxygène en mm Hg | 15,3 | 17,8 | 14,7 |
| Débit coronaire en ml/100 g.min | 70 | 104 | 56 |
| pH aortique | 7,390 | 7,355 | 7,391 |

(Suite)

| Paramètre | Essai | | |
|---|---|---|---|
| | 4 | 5 | 6 |
| Concentration plasmatique artérielle en 3-hydroxybutanoate en mM °/$_{oo}$ | 0,02 | 0,02 | 1,95 |
| Consommation par le myocarde | | | |
| – lactate en $\mu$ mol/100 g.min | 28,6 | 37,0 | 19,8 |
| – glucose en $\mu$ mol/100 g.min | 22,4 | 21,7 | 4,6 |
| – acides gras libres en $\mu$ mol/100 g.min | 2,4 | 10,7 | 1,8 |
| – 3-hydroxybutanoate en $\mu$ mol/100 g.min | 0,2 | 0,3 | 24,1 |

La comparaison des résultats du Tableau IV montre que l'administration du sel d'arginine de l'acide D(—)-3-hydroxybutanoïque (essai 6) permet de réduire la consommation propre en oxygène du myocarde soumis à des stimulations adrénergiques en dessous des valeurs mesurées avec et sans stimulation adrénergique (comparaison des essais 4 et 5) ainsi que d'augmenter le rapport entre la pression aortique et la consommation en oxygène.

Les résultats montrent également que l'assimilation des acides gras libres est régularisée et que le pH sanguin est maintenu à des valeurs normales (essais 4, 5 et 6).

En outre, la comparaison des valeurs des Tableaux III et IV (essais 3 et 6) montre que les sels dérivés de l'arginine ont comme avantage sur les sels de sodium de stabiliser la pression aortique à des valeurs identiques à celles mesurées en l'absence de stimulation adrénergique (essais 1 et 4).

### Exemple 4

Essais 7 à 9

On répète les essais décrits à l'exemple 2 mais en travaillant avec 10 chiens d'un poids corporel moyen d'environ 29 kg et avec une stimulation effectuée à l'aide d'une infusion de noradrénaline moyenne de 500 ng/kg·min dans la cavité ventriculaire gauche pendant 20 min et une administration intraveineuse de 80 micromoles/kg·min de sel de L-arginine de l'acide D(—)-3-hydroxybutanoïque de l'exemple 1. La moyenne des 10 valeurs mesurées au cours de l'essai (essai 9) et au cours d'essais de comparaison réalisés en l'absence de stimulation et de traitement (essai 7) et en effectuant la stimulation mais pas le traitement (essai 8) sont mentionnés au Tableau V ci-après.

Tableau V

| Paramètre | Essai (moyenne de 10 valeurs) | | |
|---|---|---|---|
| | 7 | 8 | 9 |
| Pression aortique en mm Hg | 124 | 141 | 136 |
| Consommation en oxygène du myocarde en ml/100 g.min | 8,5 | 12,3 | 8,9 |

**0 087 192**

(Suite)

| Paramètre | Essai (moyenne de 10 valeurs) | | |
|---|---|---|---|
| | 7 | 8 | 9 |
| Pression partielle du sang veineux coronaire en oxygène en mm Hg | 20,3 | 21,4 | 21,0 |
| Débit coronaire en ml/100 g.min | 79 | 96 | 79 |
| pH aortique | 7,396 | 7,333 | 7,344 |
| Concentration plasmatique artérielle en 3-hydroxybutanoate en mM °/₀₀ | 0,04 | 0,11 | 8,30 |
| Consommation par le myocarde | | | |
| – lactate en $\mu$mol/100 g.min | 12,0 | 4,9 | 2,2 |
| – glucose en $\mu$mol/100 g.min | 14,4 | 4,7 | 2,8 |
| – acides gras libres en $\mu$mol/100 g.min | 4,4 | 16,9 | 5,5 |
| – 3-hydroxybutanoate en $\mu$mol/100 g.min | 0,7 | 1,8 | 35,8 |

La comparaison des résultats du Tableau V montre que l'administration du sel d'arginine de l'acide D(—)-3-hydroxybutanoïque (essai 9) permet de réduire la consommation propre en oxygène du myocarde soumis à des stimulations adrénergiques par rapport aux valeurs mesurées sous stimulation adrénergique mais sans administration de l'acide D(—)-3-hydroxybutanoïque (comparaison avec l'essai 8).

Exemple 5 — Préparation de compositions pharmaceutiques

Essai 1 : En tablettes

| | |
|---|---|
| Sel de sodium de l'acide D(—)-3-hydroxybutanoïque | 500 mg |
| Sucre de lait | 170 mg |
| Fécule d'amidon | 110 mg |
| Acide silicique colloïdal | 10 mg |
| Stéarate de magnésium | 10 mg |
| | 800 mg |

La préparation se fait en mélangeant le sel de sodium de l'acide D(—)-3-hydroxybutanoïque avec une partie des adjuvants et granulation en présence d'une solution aqueuse amidonnée. Après séchage des granules le reste des adjuvants est ajouté et le mélange est comprimé en tablettes.

Essai 2 — En solution pour administration par voie orale

| | |
|---|---|
| Sel d'arginine de l'acide D(—)-3-hydroxybutanoïque | 20 g |
| Esprit de menthe | 0,4 g |
| Saccharine | 0,1 g |
| Eau distillée | 400 g |
| | 420.5 g |

Le sel d'arginine de l'acide D(—)-3-hydroxybutanoïque est mélangé avec l'esprit de menthe et la saccharine et le tout est dissous dans l'eau et introduit dans des flacons de 500 cm³.

Essai 3 : Ampoules pour injections

| | |
|---|---|
| Sel de sodium de l'acide D(—)-3-hydroxybutanoïque | 20 mg |
| Eau bidistillée | 2 g |
| | 2,2 g |

Le sel de sodium est dissous dans l'eau bidistillée et cette solution est introduite de façon stérile dans les ampoules.

Essai 4 — Solution à perfusion par voie intraveineuse

| | |
|---|---|
| Sel d'arginine de l'acide D(—)-3-hydroxybutanoïque | 70 g |
| Eau distillée | 930 g |
| | 1 000 g |

Le sel d'arginine est dissous dans l'eau distillée et la solution est introduite de façon stérile dans des poches à soluté de 1 000 cm³.

**Revendications** (pour les Etats contractants : BE, CH, DE, FR, GB, IT, LI, LU, NL, SE)

1. Compositions pharmaceutiques pour la protection métabolique du myocarde caractérisées en ce qu'elles contiennent un sel dérivé de l'acide 3-hydroxybutanoïque et d'un cation métallique dérivé des groupes Ia, IIa, et IIb du tableau périodique des éléments ou d'une base organique azotée telle qu'un acide aminé.

2. Compositions selon la revendication 1 caractérisées en ce que l'acide 3-hydroxybutanoïque est présent dans le sel sous la forme isomérique D(—).

3. Composés dérivés de l'acide 3-hydroxybutanoïque caractérisées en ce qu'ils sont des sels dérivés d'un acide aminé.

4. Composés selon la revendication 3 caractérisés en ce que l'acide aminé est un acide aminé d'origine naturelle.

5. Composés selon la revendication 4 caractérisés en ce que l'acide aminé d'origine naturelle comprend au moins deux fonctions azotées par fonction carboxylique.

6. Composés selon la revendication 4 caractérisés en ce que l'acide aminé est la L-arginine.

7. Composés selon la revendication 4 caractérisés en ce que l'acide aminé est la L-lysine ou la L-histidine.

8. Composés selon l'une quelconque des revendications 3 à 7 caractérisés en ce qu'ils sont dérivés de la forme isomérique D(—) de l'acide 3-hydroxybutanoïque.

9. Composés selon l'une quelconque des revendications 3 à 8 caractérisés en ce qu'ils sont utilisés comme médicament.

10. Compositions pharmaceutiques caractérisées en ce qu'elles contiennent un composé selon l'une quelconque des revendications 3 à 8.

11. Compositions selon la revendication 10 caractérisées en ce qu'elles comprennent en outre des additifs de formulation et/ou d'autres principes actifs vis-à-vis des affections du système cardiaque ou de la circulation sanguine.

12. Compositions pharmaceutiques à usage interne pour la protection métabolique du myocarde caractérisées en ce qu'elles contiennent un sel dérivé de l'acide 3-hydroxybutanoïque de la forme isomérique D(—) et d'un cation métallique dérivé des groupes Ia, IIa, et IIb du tableau périodique des éléments ou d'une base organique azotée telle qu'un acide aminé.

13. Compositions selon les revendications 12 caractérisées en ce qu'elles contiennent en outre des additifs de formulation et/ou d'autres principes actifs vis-à-vis des affections du système cardiaque ou de la circulation sanguine.

14. Procédé pour l'obtention des composés selon la revendication 3 caractérisés en ce que l'on fait réagir de l'acide 3-hydroxybutanoïque avec un acide aminé.

15. Utilisation d'un sel dérivé de l'acide 3-hydroxybutanoïque et d'un cation métallique dérivé des groupes Ia, IIa et IIb du tableau périodique des éléments ou d'une base organique azotée telle qu'un acide aminé pour la fabrication de produits pharmaceutiques pour le traitement ou la prévention d'affections du métabolisme du myocarde.

16. Utilisation de l'acide 3-hydroxybutanoïque pour la fabrication de produits pharmaceutiques pour le traitement ou la prévention d'affections du métabolisme du myocarde.

17. Utilisation des composés selon les revendications 3 à 8 pour la fabrication de produits pharmaceutiques.

**Revendications** (pour l'Etat contractant AT)

1. Procédé pour l'obtention de sels dérivés de l'acide 3-hydroxybutanoïque et d'un acide aminé caractérisé en ce que l'on fait réagir l'acide 3-hydroxybutanoïque et l'acide aminé dans un solvant tel que l'eau à une température comprise entre 20 et 80 °C.

2. Procédé selon la revendication 1 caractérisé en ce que l'acide aminé mis en œuvre est un acide aminé d'origine naturelle.

3. Procédé selon la revendication 2 caractérisé en ce que l'acide aminé d'origine naturelle mis en œuvre comprend au moins deux fonctions azotées par fonction carboxylique.

4. Procédé selon la revendication 3 caractérisé en ce que l'acide aminé mis en œuvre est la L-arginine.

5. Procédé selon la revendication 4 caractérisé en ce que l'acide aminé mis en œuvre est la L-lysine ou la L-histidine.

6. Procédé selon l'une quelconque des revendications 1 à 5 caractérisé en ce que l'on met en œuvre de l'acide 3-hydroxybutanoïque de la forme isomérique D(—).

7. Utilisation de l'acide 3-hydroxybutanoïque ou d'un sel dérivé de cet acide avec un cation métallique dérivé des groupes Ia, IIa et IIb du tableau périodique des éléments ou avec une base organique azotée telle qu'un acide aminé pour la fabrication de produits pharmaceutiques pour le traitement ou la prévention d'affections du métabolisme du myocarde.

8. Utilisation des composés obtenus selon les procédés des revendications 1 à 6 pour la fabrication de produits pharmaceutiques.


**Claims** (for the contracting States : BE, CH, DE, FR, GB, IT, LI, LU, NL, SE)

1. Pharmaceutical compositions for the metabolic protection of the myocardium, characterized in that they contain a salt derived from 3-hydroxybutanoic acid and from a metallic cation derived from groups Ia, IIa and IIb of the Periodic Table of Elements or from an organic nitrogen base such as an amino acid.

2. Compositions according to Claim 1, characterized in that the 3-hydroxybutanoic acid is present in the salt in the D(—) isomeric form.

3. Compounds derived from 3-hydroxybutanoic acid, characterized in that they are salts derived from an amino acid.

4. Compounds according to Claim 3, characterized in that the amino acid is an amino acid of natural origin.

5. Compounds according to Claim 4, characterized in that the amino acid of natural origin contains at least two nitrogen groups per carboxyl group.

6. Compounds according to Claim 4, characterized in that the amino acid is L-arginine.

7. Compounds according to Claim 4, characterized in that the amino acid is L-lysine or L-histidine.

8. Compounds according to any one of Claims 3 to 7, characterized in that they are derived from the D(—) isomeric form of 3-hydroxybutanoic acid.

9. Compounds according to any one of Claims 3 to 8, characterized in that they are used as a drug.

10. Pharmaceutical compositions, characterized in that they contain a compound according to any one of Claims 3 to 8.

11. Compositions according to Claim 10, characterized in that they moreover comprise formulation additives and/or other principles which are active against disorders of the cardiac system or of the blood circulation.

12. Pharmaceutical compositions for internal use for the metabolic protection of the myocardium, characterized in that they contain a salt derived from 3-hydroxybutanoic acid in the D(—) isomeric form and from a metallic cation derived from groups Ia, IIa and IIb of the Periodic Table of Elements or from an organic nitrogen base such as an amino acid.

13. Compositions according to Claims 12, characterized in that they moreover contain formulation additives and/or other principles which are active against disorders of the cardiac system or of the blood circulation.

14. Process for obtaining the compounds according to Claim 3, characterized in that 3-hydroxybutanoic acid is reacted with an amino acid.

15. Use of a salt derived from 3-hydroxybutanoic acid and from a metallic cation derived from groups Ia, IIa and IIb of the Periodic Table of Elements or from an organic nitrogen base such as an amino acid for the preparation of pharmaceutical products which are designed for the treatment or the prevention of disorders of the metabolism of the myocardium.

16. Use of 3-hydroxybutanoic acid for the preparation of pharmaceutical products which are designed for the treatment or the prevention of disorders of the metabolism of the myocardium.

17. Use of the compounds according to Claims 3 to 8 for the preparation of pharmaceutical products.


**Claims** (for the Contracting State AT)

1. Process for obtaining salts derived from 3-hydroxybutanoic acid and from an amino acid,

characterized in that 3-hydroxybutanoic acid is reacted with the amino acid in a solvent such as water at a temperature of between 20 and 80 °C.

2. Process according to Claim 1, characterized in that the amino acid used is an amino acid of natural origin.

3. Process according to Claim 2, characterized in that the amino acid of natural origin used contains at least two nitrogen groups per carboxyl group.

4. Process according to Claim 3, characterized in that the amino acid used is L-arginine.

5. Process according to Claim 4, characterized in that the amino acid used is L-lysine or L-histidine.

6. Process according to any one of Claims 1 to 5, characterized in that the 3-hydroxybutanoic acid used is in the D(—) isomeric form.

7. Use of the 3-hydroxybutanoic acid or of a salt derived from this acid with a metallic cation derived from groups Ia, IIa and IIb of the Periodic Table of Elements or with an organic nitrogen base such as an amino acid for the preparation of pharmaceutical products which are designed for the treatment or the prevention of disorders of the metabolism of the myocardium.

8. Use of the compounds obtained according to the processes of Claims 1 to 6 for the preparation of pharmaceutical products.

**Patentansprüche** (für die Vertraggstaaten : BE, CH, DE, FR, GB, IT, LI, LU, NL, SE)

1. Pharmazeutische Zusammensetzungen zum Schutz des Myocardmetabolismus, dadurch gekennzeichnet, daß sie ein Salz enthalten, das von der 3-Hydroxybutansäure und einem metallischen Kation, das von den Gruppen Ia, IIa und IIb des Periodensystems der Elemente abstammt, oder einer stickstoffhaltigen organischen Base, wie einer Aminosäure abstammt.

2. Zusammensetzungen nach Anspruch 1, dadurch gekennzeichnet, daß die 3-Hydroxybutansäure in dem Salz in der D(—)-isomeren Form vorliegt.

3. Verbindungen, die von der 3-Hydroxybutansäure abstammen, dadurch gekennzeichnet, daß sie Salze sind, die von einer Aminosäure abstammen.

4. Verbindungen nach Anspruch 3, dadurch gekennzeichnet, daß die Aminosäure eine Aminosäure natürlichen Ursprungs ist.

5. Verbindungen nach Anspruch 4, dadurch gekennzeichnet, daß die Aminosäure natürlichen Ursprungs mindestens zwei stickstoffhaltige Funktionen pro Carboxyl-Funktion umfaßt.

6. Verbindungen nach Anspruch 4, dadurch gekennzeichnet, daß die Aminosäure l-Arginin ist.

7. Verbindungen nach Anspruch 4, dadurch gekennzeichnet, daß die Aminosäure L-Lysin oder L-Histidin ist.

8. Verbindungen nach einem der Ansprüche 3 bis 7, dadurch gekennzeichnet, daß sie von der D(—)-isomeren Form der 3-Hydroxybutansäure abstammen.

9. Verbindungen nach einem der Ansprüche 3 bis 8, dadurch gekennzeichnet, daß sie als Medikament verwendet werden.

10. Pharmazeutische Zusammensetzungen, dadurch gekennzeichnet, daß sie eine Verbindung nach einem der Ansprüche 3 bis 8 enthalten.

11. Zusammensetzungen nach Anspruch 10, dadurch gekennzeichnet, daß sie außerdem Formulierungsadditive und/oder andere Bestandteile umfassen, die gegenüber krankhaften Zuständen des zum Herzen gehörenden Systems oder des Blutkreislaufs wirksam sind.

12. Pharmazeutische Zusammensetzungen für die innere Anwendung zum Schutz des Myocardmetabolismus, dadurch gekennzeichnet, daß sie ein Salz enthalten, das von der 3-Hydroxybutansäure der D(—)-isomeren Form und einem metallischen Kation, das von den Gruppen Ia, IIa und IIb des Periodensystems der Elemente abstammt, oder einer organischen stickstoffhaltigen Base, wie einer Aminosäure, abstammt.

13. Zusammensetzungen nach Anspruch 12, dadurch gekennzeichnet, daß sie außerdem Formulierungsadditive und/oder andere Bestandteile, die gegenüber krankhaften Zuständen des zum Herzen gehörenden Systems oder des Blutkreislaufs wirksam sind, enthalten.

14. Verfahren zur Herstellung der Verbindungen nach Anspruch 3, dadurch gekennzeichnet, daß man die 3-Hydroxybutansäure mit einer Aminosäure reagieren läßt.

15. Verwendung eines Salzes, das von der 3-Hydroxybutansäure und einem metallischen Kation, das von den Gruppen Ia, IIa und IIb des Periodensystems der Elemente abstammt, oder einer organischen stickstoffhaltigen Base, wie einer Aminosäure, herstammt, zur Herstellung von pharmazeutischen Produkten zur Behandlung oder Vorbeugung von krankhaften Zuständen des Myocardmetabolismus.

16. Verwendung der 3-Hydroxybutansäure zur Herstellung von pharmazeutischen Produkten zur Behandlung oder Vorbeugung von krankhaften Zuständen des Myocardmetabolismus.

17. Verwendung von Verbindungen nach den Ansprüchen 3 bis 8 zur Herstellung von pharmazeutischen Produkten.

**Patentansprüche** (für den Vertragsstaat AT)

1. Verfahren zur Herstellung von Salzen, die von der 3-Hydroxybutansäure und einer Aminosäure herstammen, dadurch gekennzeichnet, daß man die 3-Hydroxybutansäure und die Aminosäure in einem Lösungsmittel wie Wasser bei einer Temperatur zwischen 20 und 80 °C reagieren läßt.

2. Verfahren nach Anspruch 1, dadurch gekennzeichnet, daß die eingesetzte Aminosäure eine Aminosäure natürlichen Ursprungs ist.

3. Verfahren nach Anspruch 2, dadurch gekennzeichnet, daß die eingesetzte Aminosäure natürlichen Ursprungs mindestens zwei stickstoffhaltige Funktionen pro Carboxyl-Funktion umfaßt.

4. Verfahren nach Anspruch 3, dadurch gekennzeichnet, daß die eingesetzte Aminosäure L-Arginin ist.

5. Verfahren nach Anspruch 4, dadurch gekennzeichnet, daß die eingesetzte Aminosäure L-Lysin oder L-Histidin ist.

6. Verfahren nach einem der Ansprüche 1 bis 5, dadurch gekennzeichnet, daß man die 3-Hydroxybutansäure in der D(—)-isomeren Form einsetzt.

7. Verwendung der 3-Hydroxybutansäure oder eines Salzes, das von dieser Säure mit einem metallischen Kation, das von den Gruppen Ia, IIa und IIb des Periodensystems der Elemente abstammt, oder mit einer organischen stickstoffhaltigen Base, wie einer Aminosäure abstammt, zur Herstellung von pharmazeutischen Produkten zur Behandlung oder Vorbeugung von krankhaften Zuständen des Myocardmetabolismus.

8. Verwendung der Verbindungen, die nach den Verfahren der Ansprüche 1 bis 6 erhalten wurden, zur Herstellung von pharmazeutischen Produkten.